# EUROPÄISCHE PATENTSCHRIFT

(11) **EP 2 782 574 B1**
(45) Veröffentlichungstag und Bekanntmachung des Hinweises auf die Patenterteilung: **30.03.2016**
(21) Anmeldenummer: 12788550.7
(22) Anmeldetag: 22.11.2012
(51) Int. Cl.: A61K 31/4439, C07D 401/06, A61P 7/02

(54) **SALZ VON (R)-3-(6-AMINO-PYRIDIN-3-YL)-2-(1-CYCLOHEXYL-1H-IMIDAZOL-4-YL)- PROPIONSÄUREETHYLESTER**
SALT OF (R) ETHYL-3-(6-AMINO-PYRIDIN-3-YL)-2-(1-CYCLOHEXYL-1H-IMIDAZOL-4-YL)-PROPANOATE
SEL DU (R) ETHYL-3-(6-AMINO-PYRIDIN-3-YL)-2-(1-CYCLOHEXYL-1H-IMIDAZOL-4-YL)-PROPANOATE

(30) Priorität: 25.11.2011 EP 11306560
(43) Veröffentlichungstag der Anmeldung: 01.10.2014
(73) Patentinhaber: SANOFI, 75008 Paris (FR)
(72) Erfinder: ROSSEN, Kai, 65926 Frankfurt am Main (DE); WEHLAN, Hermut, 65926 Frankfurt am Main (DE)
(74) Vertreter: Then, Johann
(86) Internationale Anmeldenummer: PCT/EP2012/073295
(87) Internationale Veröffentlichungsnummer: WO 2013/076177

(56) Entgegenhaltungen:
- WO-A1-2005/105781
- MORISSETTE SHERRY L ET AL: "HIGH-THROUGHPUT CRYSTALLIZATION: POLYMORPHS, SALTS, CO-CRYSTALS AND SOLCATES OF PHARMACEUTICAL SOLIDS", ADVANCED DRUG DELIVERY REVIEWS, ELSEVIER BV, AMSTERDAM, NL, Bd. 56, Nr. 3, 1. Januar 2004 (2004-01-01) , Seiten 275-300, XP009072233, ISSN: 0169-409X, DOI: 10.1016/J.ADDR.2003.10.020
- BYRN S ET AL: "PHARMACEUTICAL SOLIDS: A STRATEGIC APPROACH TO REGULATORY CONSIDERATIONS", PHARMACEUTICAL RESEARCH, KLUWER ACADEMIC PUBLISHERS, NEW YORK, NY, US, Bd. 12, Nr. 7, 1. Juli 1995 (1995-07-01), Seiten 945-954, XP000996386, ISSN: 0724-8741, DOI: 10.1023/A:1016241927429

## Beschreibung

Die Erfindung betrifft das Salz der Naphthalen-1,5-disulfonsäure mit (R)-3-(6-Amino-pyridin-3-yl)-2-(1-cyclohexyl-1H-imidazol-4-yl)-propionsäureethylester und das Naphthalen-1,5-disulfonsäure Salz des (R)-3-(6-Amino-pyridin-3-yl)-2-(1-cyclohexyl-1 H-imidazol-4-yl)-propionsäureethylesters, welches in einer kristallinen Form oder wenigstens in einer teilweise kristallinen Form vorliegt, Verfahren zu ihrer Herstellung, diese Verbindungen enthaltende Arzneimittel und deren Verwendung, wobei die Struktur darstellbar ist durch folgende Strukturformel der Formel (I): 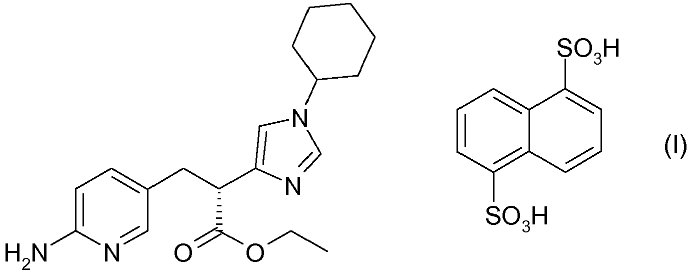 3-(6-Amino-pyridin-3-yl)-2-(1-cyclohexyl-1 H-imidazol-4-yl)-propionsäureethylesterhydrochloride und ihre pharmakologischen Eigenschaften sind bereits in der Internationalen Anmeldung PCT/EP2005/003630 (WO2005/105781) beschrieben. Die Hydrochloridsalze von 3-(6-Amino-pyridin-3-yl)-2-(1-cyclohexyl-1 H-imidazol-4-yl)-propionsäureethylester oder (R)-3-(6-Amino-pyridin-3-yl)-2-(1-cyclohexyl-1H-imidazol-4-yl)-propionsäureethylester haben den Nachteil, dass sie amorph sind und nicht kristallin erhalten werden können. Die obigen Hydrochloridsalze können daher nicht durch Kristallisation gereinigt werden und sind damit kaum brauchbar für den Einsatz als Wirkstoff in Arzneimitteln, für die vom Gesetzgeber genau definierte Reinheitsgrade der Inhaltsstoffe vorgeschrieben sind. Auch sind die armorphen Hydrochlorid-Salze aufgrund ihrer physikalischen Eigenschaften und ihrer Handhabbarkeit für die galenische Herstellung von pharmazeutischen Zubereitungen wie Tabletten nur wenig geeignet. Ferner ist (R)-3-(6-Amino-pyridin-3-yl)-2-(1-cyclohexyl-1 H-imidazol-4-yl)-propionsäureethylester unter erhöhter Temperatur und Luftfeuchtigkeit chemisch nicht stabil. Es kommt zum chemischen Abbau der Verbindung und die enatiomere Reinheit der chiralen Verbindung (R)-3-(6-Amino-pyridin-3-yl)-2-(1-cyclohexyl-1 H-imidazol-4-yl)-propionsäureethylester nimmt über die Zeit schnell ab.

Aufgabe der vorliegenden Erfindung ist es daher, die Verbindung (R)-3-(6-Amino-pyridin-3-yl)-2-(1-cyclohexyl-1 H-imidazol-4-yl)-propionsäureethylester in einer geeigneten Form bereitzustellen, die leicht zu reinigen ist und eine verbesserte Stabilität bei erhöhter Temperatur und Luftfeuchtigkeit aufweist.

Die vorliegende Erfindung betrifft somit das Salz der Naphthalen-1,5-disulfonsäure mit (R)-3-(6-Amino-pyridin-3-yl)-2-(1-cyclohexyl-1H-imidazol-4-yl)-propionsäureethylester, das in kristalliner Form oder in teilweiser kristalliner Form vorliegen kann. Die vorliegende Erfindung betrifft auch das Salz der Verbindung der Formel (I) 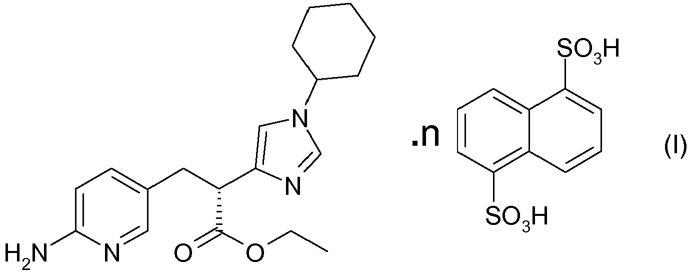 worin n einen Wert von 0,5 bis 1,8 besitzt und n das molare Verhältnis von (R)-3-(6-Amino-pyridin-3-yl)-2-(1-cyclohexyl-1H-imidazol-4-yl)-propionsäureethylester zu Naphthalen-1,5-disulfonsäure angibt.

Bevorzugt sind Salze der Verbindung der Formel I, worin n einen Wert von 0,8 bis 1,3 besitzt.

Bevorzugt sind Salze der Verbindung der Formel I, worin n einen Wert von 0,95 bis 1,05 besitzt.

Das erfindungsgemäße Salz, insbesondere das Salz der Verbindung der Formel (I) ist zumindest teilweise, vorzugsweise vollständig kristallin. Durch die Bereitstellung der erfindungsgemäßen insbesondere vollständig kristallinen Salze der Verbindung der Formel (I) kann der Wirkstoff (R)-3-(6-Amino-pyridin-3-yl)-2-(1-cyclohexyl-1 H-imidazol-4-yl)-propionsäureethylester
- leichter gereinigt werden (z.B. durch Umkristallisieren)
- eine für die Arzneimittelzulassung nötige definierte Reinheit aufweisen
- leicht durch gängige Methoden wie XRPD (X-ray Powder Diffraction; Röntgenpulverdiffraktometrie), Schmelzpunkt, IR (Infrarot Spektrum) nachgewiesen und identifiziert werden und er besitzt
- eine reproduzierbare physikalische Qualität
- eine verbesserte chemische Stabilität und
- eine verbesserte chirale Stabilität

Kristalline Wirkstoffe sind in der Regel stabiler als amorphe. Dadurch werden Probleme mit dem Abbau der Wirkstoffe und den auftretenden Abbauprodukten vermieden.

Die amorphe Form eines Wirkstoffs kann noch einen ungewünschten Gehalt an Lösungsmitteln enthalten. Diese sind in der Regel schwer zu entfernen, da nicht umkristallisiert werden kann.

Die amorphe Form ist energiereicher als die kristalline Form. Das kann dazu führen, dass sich das zufällige Muster der Molekülverteilung der amorphen Form spontan unter Energieabgabe rearrangiert und einen Teil der Energie abführt. Dies kann zu einer Änderung der Wirkung des Wirkstoffs führen, ohne dass dies direkt an einem messbaren Parameter des Wirkstoffs sichtbar würde. Ein signifikanter Einfluss auf die Zuverlässigkeit des Wirkstoffs und somit ein Risiko für den Patienten ist die Folge. Nur ein definierter kristalliner Wirkstoff erlaubt eine zuverlässige und reproduzierbare Formulierung mit reproduzierbarer Bioverfügbarkeit.

Eine weitere Ausführungsform der Erfindung umfasst kristallines Salz der Formel I, dadurch gekennzeichnet, dass das mit CuKα-Strahlung gemessene XRPD bei einer Winkellage von 22,48 Grad 2 Theta ± 0,2 Grad 2 Theta einen Reflex mit hoher Intensität aufweist.

Eine weitere Ausführungsform der Erfindung umfasst kristallines Salz der Formel I, dadurch gekennzeichnet, dass das mit CuKα-Strahlung gemessene XRPD bei den Winkellagen Reflexe bei den folgenden 2 Theta Werten aufweist:
7,35 und 22,48 jeweils ± 0,2 Grad 2 Theta.

Eine weitere Ausführungsform der Erfindung umfasst kristallines Salz der Formel I, dadurch gekennzeichnet, dass das mit CuKα-Strahlung gemessene XRPD bei den Winkellagen Reflexe bei den folgenden 2 Theta Werten aufweist:
7,35 ; 11,4 ; 13,69 ; 14,96; 17,49 ; 19,3 und 22,48 jeweils ± 0.2 Grad 2 Theta.

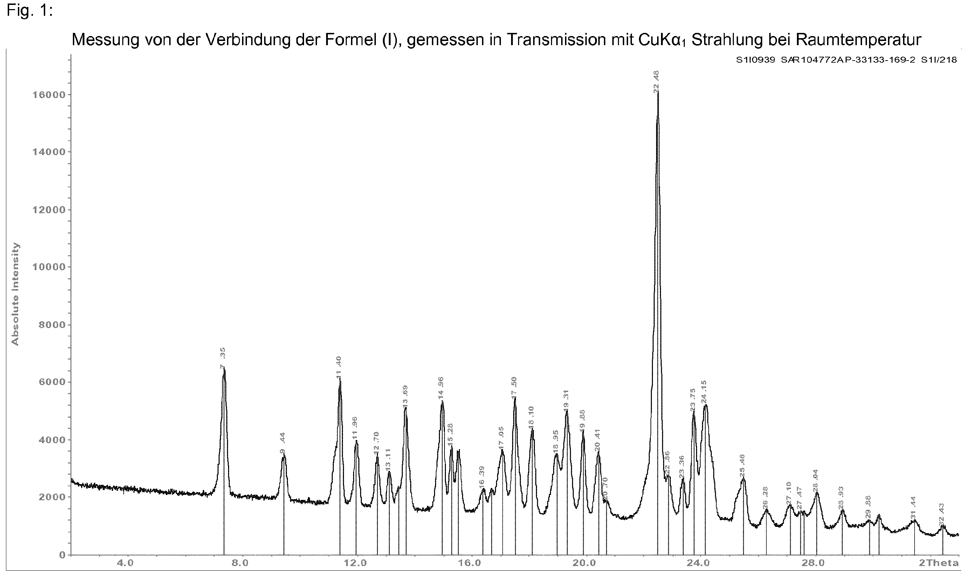

Die Auswahl der charakteristischen Reflexe erfolgte aufgrund der Höhe der Intensität. Ein Beispiel für ein XRPD ist in Fig. 1 wiedergegeben.

Die erfindungsgemäße Ausführungsform kann auch noch in Abhängigkeit von der relativen Luftfeuchtigkeit eine geringe Menge von Wasser enthalten; der Wassergehalt beträgt von etwa 0,1 % bis 2,0 %. Die Prozentangaben beziehen sich jeweils auf das Gewicht. Dieses Wasser lässt sich aus dem kristallinen Salz der Formel (I) beim Erhitzen auf über 100 °C weitestgehend vollständig entfernen. XRPD Messungen bei verschiedenen Temperaturen zeigen im wesentlichen unveränderte Spektren.

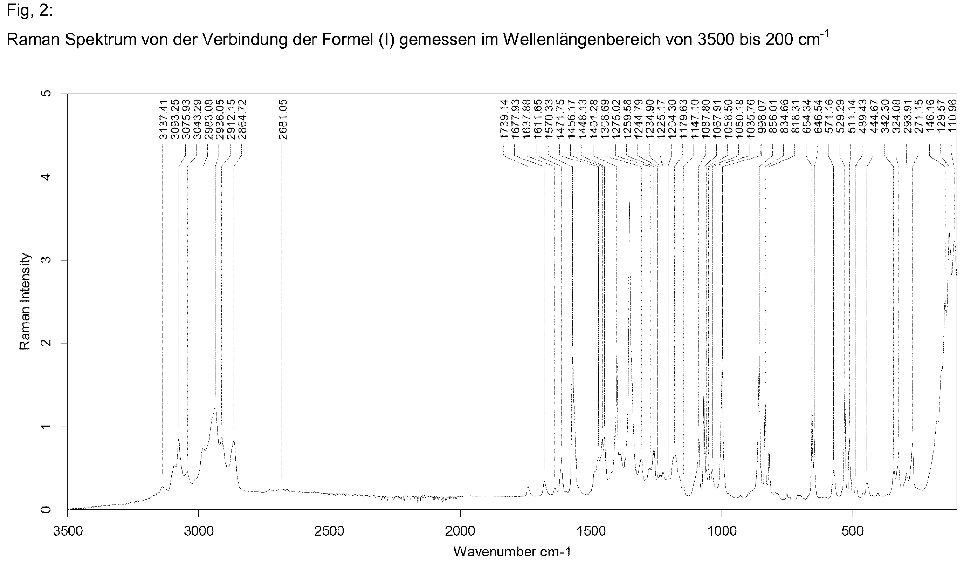

Alternativ lässt sich das kristalline Salze von Naphthalen-1,5-disulfonsäure mit (R)-3-(6-Amino-pyridin-3-yl)-2-(1-cyclohexyl-1H-imidazol-4-yl)-propionsäureethylester der Formel (I) auch durch ein Raman-Spektrum charakterisieren. Dabei lassen sich charakteristische Banden bei den folgenden Wellenlängen [cm⁻¹] bestimmen: 2936; 1570; 1401; 1352; 998 und 856 jeweils ± 2 cm⁻¹
Ein Beispiel für ein Raman-Spektrum ist in Fig. 2 wiedergegeben.

Unter dem Begriff "Polymorphismus" wird die Fähigkeit von einzelnen Verbindungen verstanden in mehr als einer Form oder kristallinen Struktur zu existieren. Verschiedene Polymorphe sind Feststoffe, die durch die gleiche Summenformel charakterisiert sind, aber unterschiedliche physikalische Eigenschaften haben können.
Unter dem Begriff "amorph" werden feste Verbindungen bezeichnet, die im XRPD-Spektrum bei allen Winkellagen keine Reflexe aufweisen, die sich in ihren Intensitäten deutlich voneinander abgrenzen lassen.
Unter dem Begriff "Intensität" werden die Menge der gemessenen Reflexe bezeichnet. Unter dem Begriff "charakteristische Banden" beim Ramanspektrum werden Banden bezeichnet, die der Kristallform eindeutig zugeordnet werden können.

### Beschreibung der Figuren:

Fig. 1: XRPD Spektrum vom kristallinen Salz von Naphthalen-1,5-disulfonsäure mit (R)-3-(6-Amino-pyridin-3-yl)-2-(1-cyclohexyl-1H-imidazol-4-yl)-propionsäureethylester der Formel (I), gemessen in Transmission mit CuKα₁ Strahlung bei Raumtemperatur (X-Achse Beugungswinkel 2theta (2θ) [°]; Y-Achse: relative Intensität)
Fig. 2: Raman Spektrum vom kristallinen Salz von Naphthalen-1,5-disulfonsäure mit (R)-3-(6-Amino-pyridin-3-yl)-2-(1-cyclohexyl-1H-imidazol-4-yl)-propionsäureethylester der Formel (I), gemessen im Wellenlängenbereich von 3500 bis 200 cm⁻¹ (X-Achse Wellenlänge [cm⁻¹];Y-Achse: relative Intensität)

Die Erfindung betrifft weiterhin Verfahren zur Darstellung der Verbindung der Formel (I). Ein Verfahren zur Herstellung der Verbindung der Formel (I) ist dadurch gekennzeichnet, dass man Naphthalen-1,5-disulfonsäure in einem Lösungsmittel A auflöst und eine Lösung von (R)-3-(6-Amino-pyridin-3-yl)-2-(1-cyclohexyl-1 H-imidazol-4-yl)-propionsäureethylester in einem Lösungsmittel B zufügt, anschließend wird das Salz von (R)-3-(6-Amino-pyridin-3-yl)-2-(1-cyclohexyl-1H-imidazol-4-yl)-propionsäureethylester mit Naphthalen-1,5-disulfonsäure der Formel (I) erhalten. Gegebenenfalls werden die Lösungsmittel teilweise oder vollständig entfernt. Das erhaltene Salz ist zumindest teilweise, vorzugsweise vollständig kristallin.
Geeignete Lösungsmittel A sind beispielsweise polare protische oder aprotische Lösungsmittel wie Ethanol, Aceton, Methylethylketon, Acetonitril, Tetrahydrofuran, Dimethylsulfoxid, Dimethylformamid, N-Methylpyrrolidinone, Methanol, Propanol, Butanol oder Wasser. Es können auch Mischungen von einem oder mehreren der genannten Lösungsmittel verwendet werden.

Geeignete Lösungsmittel B sind beispielsweise polare protische oder aprotische Lösungsmittel wie Ethanol, Aceton, Methylethylketon, Acetonitril, Tetrahydrofuran, Dimethylsulfoxid, Dimethylformamid, N-Methylpyrrolidinone, Methanol, Propanol, Butanol oder Wasser. Es können auch Mischungen von einem oder mehreren der genannten Lösungsmittel verwendet werden.

Die Lösungsmittel A und B können gleich oder verschieden sein.

Das Entfernen der Lösungsmittel kann beispielsweise durch Erwärmen erfolgen oder durch Überleiten oder Einleiten eines Gases. Geeignete Gase sind beispielsweise Luft, Stickstoff oder Edelgase wie Argon oder Xenon.

Vorteilhaft ist die Zugabe von (R)-3-(6-Amino-pyridin-3-yl)-2-(1-cyclohexyl-1 H-imidazol-4-yl)-propionsäureethylester in eine Lösung von Naphthalen-1,5-disulfonsäure, weil dadurch die mögliche Razemisierung von (R)-3-(6-Amino-pyridin-3-yl)-2-(1-cyclohexyl-1H-imidazol-4-yl)-propionsäureethylester weitgehend vermindert wird.

Das molare Verhältnis von Naphthalen-1,5-disulfonsäure zu (R)-3-(6-Amino-pyridin-3-yl)-2-(1-cyclohexyl-1H-imidazol-4-yl)-propionsäureethylester in dem Verfahren zur Herstellung der Verbindung der Formel (I) beträgt von 0,95 bis 1,5. Vorteilhaft ist ein Verhältnis von 1,1 bis 1,2.
Geeignete Temperaturen im Verfahren zur Herstellung der Verbindung der Formel (I) betragen von -20 °C bis 60 °C oder von -10 °C bis 25 °C. Die geeigneten Temperaturen hängen vom Lösungsmittel ab und können von einem Fachmann leicht ermittelt werden.

Die Erfindung betrifft auch die Verbindung der Formel (I) als Arzneimittel.

Die Erfindung betrifft auch Arzneimittel, gekennzeichnet durch eine Verbindung der Formel (I) zusammen mit einem pharmazeutisch geeigneten und physiologisch verträglichen Träger.

Aufgrund der pharmakologischen Eigenschaften eignen sich die erfindungsgemäßen Verbindungen zur Prophylaxe und Therapie all solcher Erkrankungen, die durch eine Hemmung von TAFla behandelbar sind. So eignet sich die Verbindung der Formel (I) sowohl für eine prophylaktische als auch für eine therapeutische Anwendung am Menschen. Sie eignen sich sowohl für eine akute Behandlung als auch für eine Langzeittherapie. Die Verbindung der Formel (I) kann eingesetzt werden in Patienten, die an Störungen des Wohlbefindens oder Krankheiten leiden, die mit Thrombosen, Embolien, Hyperkoagulabilität oder fibrotischen Veränderungen einhergehen.
Dazu gehören der Myokardinfarkt, die Angina pectoris und alle anderen Formen des akuten Koronarsyndroms, der Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung, Angioplastie und ähnlichen Eingriffen wie Stentimplantationen und Bypass-Operationen. Weiterhin kann die Verbindung der Formel (I) eingesetzt werden bei allen Eingriffen, die zu einem Kontakt des Blutes mit Fremdoberflächen führen wie bei Dialysepatienten und Patienten mit Verweilkathetern. die Verbindung der Formel (I) kann eingesetzt werden, um die Thrombosegefahr nach chirurgischen Eingriffen wie bei Knie- und Hüftgelenksoperationen zu reduzieren.

Die Verbindung der Formel (I) eignet sich für die Behandlung von Patienten mit disseminierter intravaskulärer Koagulation, Sepsis und anderen intravaskulären Ereignissen, die mit einer Entzündung einhergehen. Weiterhin eignet sich die Verbindung der Formel (I) für die Prophylaxe und Behandlung von Patienten mit Atherosklerose, Diabetes und dem metabolischen Syndrom und dessen Folgen. Störungen des hämostatischen Systems (z.B. Fibrinablagerungen) wurden impliziert in Mechanismen, die zu Tumorwachstum und Tumormetastasierung führen; die Verbindung der Formel (I) eignet sich zur Verlangsamung oder Verhinderung solcher Prozesse.

Weitere Indikationen für den Einsatz der Verbindung der Formel (I) sind fibrotische Veränderungen der Lunge wie die chronische obstruktive Lungenerkrankung, das adult respiratory distress syndrome (ARDS) und des Auges wie Fibrinablagerungen nach Augenoperationen. Die Verbindung der Formel (I) eignet sich auch zur Verhinderung und/oder Behandlung von Narbenbildung.

Die Applikation der erfindungsgemäßen Arzneimittel kann durch orale, inhalative, rektale oder transdermale Applikation oder durch subkutane, intraartikuläre, intraperitoneale oder intravenöse Injektion erfolgen. Bevorzugt ist die orale Applikation. Eine Beschichtung von Stents oder anderer Oberflächen, die im Körper mit Blut in Kontakt kommen, mit der Verbindung der Formel (I) ist möglich.

Die Erfindung betrifft auch ein Verfahren zur Herstellung eines Arzneimittels, das dadurch gekennzeichnet, dass man mindestens eine Verbindung der Formel (I) mit einem pharmazeutisch geeigneten und physiologisch verträglichen Träger in eine geeignete Darreichungsform bringt.

Zur Therapie der oben genannten Erkrankungen kann die Verbindung der Formel (I) selbst als Verbindung verwendet werden, vorzugsweise liegt sie jedoch mit einem geeigneten Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% der Verbindung der Formel (I) enthalten kann. Weitere pharmazeutisch aktive Substanzen können ebenfalls vorhanden sein. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die günstigste Verabreichungsweise in jedem Einzelfall von der Art und Schwere des zu behandelnden Zustandes abhängig ist. Auch dragierte Formulierungen und dragierte Retardformulierungen gehören in den Rahmen der Erfindung. Möglich sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung der Formel (I) enthalten; als Pulver oder Granulate; als Lösung oder Suspension in einer wässrigen oder nicht-wässrigen Flüssigkeit; oder als eine Öl-in-Wasser- oder Wasserin-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteter Verbindung in einer geeigneten Maschine hergestellt werden.
Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Weitere geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuss- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind Tagesdosen von etwa 2 mg bis 1000 mg der Verbindung der Formel (I), bevorzugt etwa 50 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die Verbindung der Formel (I) kann sowohl als Monotherapie als auch in Kombination oder gemeinsam mit Antithrombotika (Antikoagulanzien und Plättchenaggregationshemmer), Thrombolytika (Plasminogenaktivatoren jeglicher Art), anderen profibrinolytisch wirksamen Substanzen, Blutdrucksenkern, Regulatoren des Blutzuckers, Lipidsenkern und Antiarrhythmika verabreicht werden.

Nachfolgend wird die Herstellung einiger Beispiele detailliert beschrieben. Experimenteller Teil

### Beispiel 1

Herstellung des kristallinen Salzes von (R)-3-(6-Amino-pyridin-3-yl)-2-(1-cyclohexyl-1 H-imidazol-4-yl)-propionsäureethylester mit Naphthalen-1,5-disulfonsäure

1,50 g (5,20 mmol, 1,2 eq.) Naphthalen-1,5-disulfonsäure werden in 15 mL Wasser gelöst. Gegebenenfalls können auch einige Kristalle der Verbindung der Formel (I) zugegeben werden. Die Lösung wird auf 10 °C gekühlt. Anschließend werden 1,48 g (4,32 mmol, 1,0 eq.) (R)-3-(6-Amino-pyridin-3-yl)-2-(1-cyclohexyl-1 H-imidazol-4-yl)-propionsäureethylester (hergestellt wie in WO2005/105781 beschrieben), das vorher in 15 mL Aceton/Wasser 2:3 gelöst wurde, langsam zugetropft. Die Lösungsmittel werden durch Überströmen von Argon allmählich entfernt und wobei sich langsam Kristalle bilden. Der Argonstrom wird gestoppt und die Mischung wird für 3 Stunden (h) bei Raumtemperatur (20 °C bis 22 °C) gerührt. Die Feststoffe werden gefiltert und über Nacht an der Luft getrocknet. So konnte eine Ausbeute von 1,83 g (2,90 mmol, 67%) des kristallinen Salzes von (R)-3-(6-Amino-pyridin-3-yl)-2-(1-cyclohexyl-1 H-imidazol-4-yl)-propionsäureethylester mit Naphthalen-1,5-disulfonsäure als weißer Feststoff erhalten werden.

Das erhaltene kristalline Salz von (R)-3-(6-Amino-pyridin-3-yl)-2-(1-cyclohexyl-1 H-imidazol-4-yl)-propionsäureethylester mit Naphthalen-1,5-disulfonsäure zeigt das in Fig. 1 wiedergegebene XRPD. Die Reflexe bei den folgenden 2 Theta Werten weisen die größten Intensitäten auf und die relativen Intensitäten sind in Klammer dargestellt: 7,35 (40); 9,43 (21); 11,40 (37); 11,96 (24); 12,70 (21); 13,11 (17); 13,43 (14); 13,69 (31); 14,96 (33); 15,28 (23); 15,53 (22); 16,39 (14); 16,68 (14); 17,05 (22); 17,49 (33); 18,09 (27); 18,95 (21); 19,31 (30); 19,88 (26); 20,40 (22); 20,69 (11); 22,48 (100); 22,85 (17); 23,35 (16); 23,74 (30); 24,14 (32); 25,46 (16); 26,27 (9); 27,09 (10); 27,46 (9); 27,58 (9); 28,03 (13); 28,93 (9); 29,88 (7); 30,20 (8); 31,43 (7); 32,43 (6). Die 2 Theta Werte mit den höchsten Intensitäten sind in Tabelle 1 dargestellt:

**Tabelle 1:**

| | | | | | | | |
|---|---|---|---|---|---|---|---|
| 2 Theta (+/- 0,2 Grad 2 Theta) | 7,35 | 11,4 | 13,69 | 14,96 | 17,49 | 19,3 | 22,48 |

Aufgrund natürlicher Abweichungen in den Proben oder in der Meßmethode können die 2 Theta Werte der Winkellagen mit einer Genauigkeit von +/- 0,2 Grad Theta angegeben werden.

Die XRPD-Messungen erfolgen in einem STOE Stadi-P Transmissionsdiffraktometer;

| | | | |
|---|---|---|---|
| Strahlenquelle: | CuKα₁, λ = 1,5406 A | | |
| Winkelbereich: | 2° bis 40° in 2-theta Bragg | | |
| Schrittweite: | 0,033° | Schrittzeit: | 1 s |

Bei Raumtemperatur werden lineare positionsensitive Detektoren verwenden, Trockene Proben werden in einer flachen Anordnung gemessen, während Suspensionen in Quarzkapillaren gemessen werden, Die aufgenommenen Daten werden mit der Software WinXPOW V2,12 verarbeitet,

### Raman Spektroskopie

Das Raman Spektrum wird mit einem FT-Raman Spektrometer (RFS-100/S, BRUKER) ausgerüstet mit einem 1,5W NIR-Laser (Nd:YAG; λ = 1064 nm) und einem mit Stickstoff gekühlten D418-T NIR-Detektor aufgenommen, Das aufgenommene Spektrum wird mit der Software OPUS V, 4,2verarbeitet, Das erhaltene kristalline Salz von (R)-3-(6-Amino-pyridin-3-yl)-2-(1-cyclohexyl-1H-imidazol-4-yl)-propionsäureethylester mit Naphthalen-1,5-disulfonsäure zeigt das in Fig, 2 wiedergegebene Spektrum, Dabei lassen sich charakteristische Banden bei den folgenden Wellenlängen [cm ⁻¹] bestimmen:
2936; 1570; 1401; 1352; 998 und 856 jeweils ± 2 cm⁻¹

Aufgrund natürlicher Abweichungen in den Proben oder in der Meßmethode können die charakteristischen Banden bei den Wellenlängen mit einer Genauigkeit von +/- 2 cm⁻¹ angegeben werden.
Chiral HPLC: (Chiralcel OD-H/84 (250x4.6 mm), Heptan:iPrOH:MeCN 15:1:0.5 +0.1% DEA, 30°C, 1 ml/min): Rₜ = 27.01 min.
NMR (d6-DMSO, 600 MHz): δ = 1,12 (t, 3H), 1,13-1,21 (m, 1H), 1,29-1,39 (m, 2H), 1,57-1,68 (m, 3H), 1,75-1,82 (m, 2H), 1,95-2,02 (m, 2H), 3,02 (dd, 1 H), 3,15 (dd, 1 H), 4,05-4,15 (m, 2H), 4,17-4,24 (m, 2H), 6,85-6,89 (m, 1 H), 7,39-7,44 (m, 2H), 7,68-7,72 (m, 2H), 7,73-7,76 (m, 1 H), 7,88-7,99 (m, 4H), 8,86-8,90 (m, 2H), 9,08 (s, 1 H).

### Beispiel 2

### Salz-Sreening

100 mg der Verbindung (R)-3-(6-Amino-pyridin-3-yl)-2-(1-cyclohexyl-1 H-imidazol-4-yl)-propionsäureethylester (im folgenden API genannt) hergestellt wie in WO2005/105781 beschrieben, werden in 10 mL Aceton in einem Ultraschallbad bei Raumtemperatur gelöst, Das molare Verhältnis zwischen der H⁺-Konzentration der Säure und des API beträgt 2 (für Details siehe Tabelle 2), In jeder Vertiefung der Mikrotiterplatte werden 68 mikro Liter vom gelösten API mit 40 mikro Liter der 0,1 normalen Säurelösungen gemischt.

**Tabelle 2:**

| Säure | Molekular -gewicht | H+ Eq, | Masse [g] in 100 mL Lösung | Lösemittel | Molarität der Säure [mol/L] | Molarität von H⁺ [mol/L] |
|---|---|---|---|---|---|---|
| Adipinsäure | 146,14 | 2 | 0,731 | Ethanol | 0,05 | 0,1 |
| Benzoesäure | 122,12 | 1 | 1,221 | Ethanol | 0,1 | 0,1 |
| Bernsteinsäure | 118,09 | 2 | 0,590 | Wasser: Ethanol 1:1 | 0,05 | 0,1 |
| Zitronensäure | 192,13 | 2 | 0,961 | Wasser: Ethanol 1:1 | 0,05 | 0,1 |
| Essigsäure | 60,05 | 1 | 0,601 | Ethanol | 0,1 | 0,1 |
| Fumarsäure | 116,08 | 2 | 0,580 | Methanol | 0,05 | 0,1 |
| Glucuronsäure | 194,14 | 1 | 1,941 | Wasser: Ethanol 3:1 | 0,1 | 0,1 |
| Weinsäure | 150,09 | 2 | 0,750 | Wasser: Ethanol 1:1 | 0,05 | 0,1 |
| Phosphorsäure | 98,00 | 1 | 0,980 | Ethanol | 0,1 | 0,1 |
| Malonsäure | 104,06 | 2 | 0,520 | Wasser: | 0,05 | 0,1 |
| | | | | Ethanol 1:1 | | |
| Salzsäure | 36,46 | 1 | 0,365 | Ethanol | 0,1 | 0,1 |
| Schwefelsäure | 98,08 | 2 | 0,490 | Ethanol | 0,05 | 0,1 |

| | | | | | | |
|---|---|---|---|---|---|---|
| Eq, bedeutet Äquivalente | | | | | | |

### Versuchsdurchführung:

In jede Vertiefung einer 96-Mikrotiterplatte werden 68 mikro Liter des gelösten API pipettiert, Es werden dann je 40 mikro Liter der wie in Tabelle 2 gelösten Säuren gemäß dem Schema wie in Tabelle 3 ausgeführt zugefügt, Die Mikrotiterplatte wird bei Raumtemperatur für 20 min geschüttelt, so dass sich ein Gleichgewicht in den Vertiefungen ausbildet, Verdampfung der Lösemittel bei Raumtemperatur in einem Strom von Stickstoff für 2 Stunden, Dann werden je 40 mikro Liter der Lösemittel gemäß dem Schema wie in Tabelle 3 ausgeführt zugefügt, Die Mikrotiterplatte wird bei Raumtemperatur für 20 min geschüttelt, so dass sich ein Gleichgewicht in den Vertiefungen ausbildet, Dann erfolgt das Verdampfen der Lösemittel bei Raumtemperatur in einem Strom von Stickstoff für 2 Stunden, Abschließend erfolgt die XRPD Messung, Die Ergebnisse werden in Tabelle 3 gezeigt,

**Tabelle 3:**

| | Wasser | Wasser: Methanol 1:1 | Methanol | Ethanol | 2-Propanol | 2-Methyl -1-propanol | Methyl -ethylketon | Butylacetat |
|---|---|---|---|---|---|---|---|---|
| Salzsäure | NKP | NKP | NKP | NKP | NKP | NKP | NKP | NKP |
| Schwefelsäure | NKP | NKP | NKP | NKP | NKP | NKP | NKP | NKP |
| Phosphorsäure | NKP | NKP | NKP | NKP | NKP | NKP | NKP | NKP |
| Essigsäure | NKP | NKP | NKP | NKP | NKP | NKP | NKP | NKP |
| Weinsäure | NKP | NKP | NKP | NKP | NKP | NKP | NKP | NKP |
| Zitronensäure | NKP | NKP | NKP | NKP | NKP | NKP | NKP | NKP |
| Fumarsäure | NKP | NKP | NKP | NKP | NKP | NKP | NKP | NKP |
| Bernsteinsäure | NKP | NKP | NKP | KFA | KFA | KFA | NKP | KFA |
| Malonsäure | NKP | NKP | NKP | NKP | NKP | NKP | NKP | NKP |
| Adipinsäure | KFA | NKP | KFA | KFA | KFA | KFA | KFA | KFA |
| Benzoesäure | KFA | NKP | KFA | KFA | KFA | KFA | KFA | KFA |
| Glucuronsäure | NKP | NKP | NKP | NKP | NKP | NKP | NKP | NKP |

| | | | | | | | | |
|---|---|---|---|---|---|---|---|---|
| NKP bedeutet keine kristalline Phase KFA bedeutet freie Säure kristallisiert | | | | | | | | |

Die Ergebnisse zeigen, dass mit den getesteten Säuren und Lösemitteln kein kristallines Salz von (R)-3-(6-Amino-pyridin-3-yl)-2-(1-cyclohexyl-1 H-imidazol-4-yl)propionsäureethylester gefunden wurde und es daher sehr schwierig ist ein festes kristallines Salz von (R)-3-(6-Amino-pyridin-3-yl)-2-(1-cyclohexyl-1 H-imidazol-4-yl)propionsäureethylester zu finden,

### Beispiel 3

### Physikalische Stabilität

Repräsentative Proben des kristallinen Salzes von (R)-3-(6-Amino-pyridin-3-yl)-2-(1-cyclohexyl-1 H-imidazol-4-yl)-propionsäureethylester mit Naphthalen-1,5-disulfonsäure hergestellt wie in Beispiel 1 werden über 14 Tage unter den Bedingungen (A), (B), (C) und (D) aufbewahrt:
(A) 50 °C
(B) 50 °C und 80 % relative Luftfeuchtigkeit
(C) 80 °C
(D) 80 °C und 80 % relative Luftfeuchtigkeit

### Ergebnisse:

Die XRPD-Messungen am Anfang und nach 14 Tagen sind nahe beieinander, Es ist nur ein geringer Masseverlust bei den Thermogravimetriemessungen (Apparat TA Instuments: TGA Q500, Atmosphäre Stickstoff; Rate 10 °C/min) zu beobachten der auf den Abgang von Wasser aus den Kristallen zurückzuführen ist, Der Masseverlust beträgt zwischen 1,01 % und 1,43 %,

### Beispiel 4

### Chirale Stabilität

Repräsentative Proben des kristallinen Salzes von (R)-3-(6-Amino-pyridin-3-yl)-2-(1-cyclohexyl-1 H-imidazol-4-yl)-propionsäureethylester mit Naphthalen-1,5-disulfonsäure hergestellt wie in Beispiel 1 werden über 14 Tage unter den in Beispiel 4 beschriebenen Bedingungen (A), (B), (C) und (D) aufbewahrt:

**Ergebnisse:**

| Lagerbedingungen | (A) | (B) | (C) | (D) |
|---|---|---|---|---|
| Enantiomere Reinheit | 98,8 % | 98,8 % | 99,0 % | 96,3 % |

Die enantiomere Reinheit der gemessenen Probe betrug am Anfang der Messung 99,0 %, Unter der Lagerbedingung 50 °C (A) und 50 °C und 80 % relative Luftfeuchtigkeit (B) konnte eine geringe Zunahme von 0,2 % des (S)-Enatiomeren beobachtet werden, Unter den Lagerbedingungen 80 °C (C) konnte keine Veränderung beobachtet werden, Unter der Lagerbedingung 80 °C und 80 % relative Luftfeuchtigkeit (D) konnte eine Zunahme von 2,7 % des (S)-Enatiomeren beobachtet werden,
Die Messungen erfolgten mit Chromatographie an chiraler Phase wie in Beispiel 1 beschrieben,
Chiral HPLC: (Chiralcel OD-H/84 (250x4.6 mm), Heptan : Isopropanol : Acetonitril 15:1:0.5 +0.1% Diethylamin (DEA), 30°C, 1 ml/min): Rₜ = 27,01 min.

## Patentansprüche

1. Naphthalen-1,5-disulfonsäure Salz von (R)-3-(6-Amino-pyridin-3-yl)-2-(1cyclohexyl-1 H-imidazol-4-yl)-propionsäure-ethylester,

2. Naphthalen-1,5-disulfonsäure Salz gemäß Anspruch 1, **dadurch gekennzeichnet, dass** das Salz in kristalliner Form oder in partiell kristalliner Form vorliegt,

3. Salz der Verbindung der Formel (I) gemäß Anspruch 1 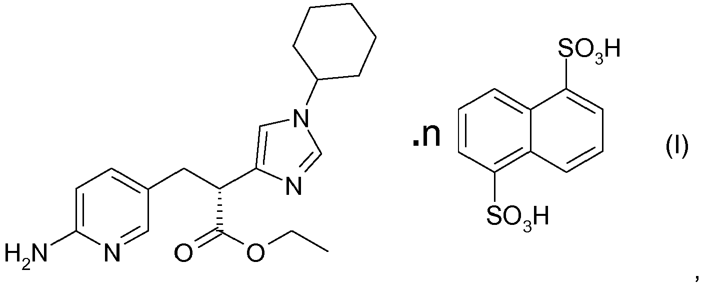 worin n einen Wert von 0,5 bis 1,8 besitzt und n das molare Verhältnis von (R)-3-(6-Amino-pyridin-3-yl)-2-(1-cyclohexyl-1H-imidazol-4-yl)-propionsäureethylester zu Naphthalen-1,5-disulfonsäure angibt,

4. Salz der Verbindung der Formel I gemäß Anspruch 3, worin n einen Wert von 0,8 bis 1,3 besitzt,

5. Salz der Verbindung der Formel I gemäß Anspruch 4, worin n einen Wert von 0,95 bis 1,05 besitzt,

6. Salz der Verbindung der Formel I gemäß Anspruch 3, **dadurch gekennzeichnet, dass** das mit CuKα-Strahlung gemessene XRPD bei einer Winkellage von 22,48 Grad 2 Theta ± 0,2 Grad 2 Theta einen Reflex mit hoher Intensität aufweist,

7. Salz der Verbindung der Formel I gemäß Anspruch 3, **dadurch gekennzeichnet, dass** das mit CuKα-Strahlung gemessene XRPD bei den Winkellagen Reflexe bei den folgenden 2 Theta Werten aufweist:
7,35 und 22,48 jeweils ± 0,2 Grad 2 Theta,

8. Salz der Verbindung der Formel I gemäß Anspruch 3, **dadurch gekennzeichnet, dass** das mit CuKα-Strahlung gemessene XRPD bei den Winkellagen Reflexe bei den folgenden 2 Theta Werten aufweist:
7,35 ; 11,4 ; 13,69 ; 14,96; 17,49 ; 19,3 und 22,48 jeweils ± 0,2 Grad 2 Theta,

9. Salz der Verbindung der Formel I gemäß Anspruch 3, **dadurch gekennzeichnet, dass** das mit CuKα-Strahlung gemessene XRPD Reflexe aufweist, die im wesentlichen identisch mit denen in Figur 1 dargestellt sind,

10. Salz der Verbindung der Formel I gemäß Anspruch 3, **dadurch gekennzeichnet, dass** das Raman-Spektrum charakteristische Banden bei den folgenden Wellenlängen [cm ⁻¹] aufweist:
2936; 1570; 1401; 1352; 998 und 856 jeweils ± 2 cm ⁻¹

11. Verfahren zur Herstellung des Salzes gemäß Anspruch 1, **dadurch gekennzeichnet, dass** man Naphthalen-1,5-disulfonsäure in einem Lösungsmittel A auflöst und eine Lösung von (R)-3-(6-Amino-pyridin-3-yl)-2-(1-cyclohexyl-1 H-imidazol-4-yl)-propionsäureethylester in einem Lösungsmittel B zufügt, anschließend wird das Salz von (R)-3-(6-Amino-pyridin-3-yl)-2-(1-cyclohexyl-1 H-imidazol-4-yl)-propionsäureethylester mit Naphthalen-1,5-disulfonsäure der Formel (I) erhalten, gegebenenfalls werden die Lösungsmittel teilweise oder vollständig entfernt,

12. Verfahren zur Herstellung gemäß Anspruch 11, **dadurch gekennzeichnet, dass** die Lösungsmittel A und B gleich oder verschieden sind und ausgewählt werden aus der Gruppe Ethanol, Aceton, Methylethylketon, Acetonitril, Tetrahydrofuran, Dimethylsulfoxid, Dimethylformamid, N-Methylpyrrolidinon, Methanol, Propanol, Butanol und Wasser,

13. Arzneimittel, **gekennzeichnet durch** ein Salz gemäß einem oder mehreren der Ansprüche 1 bis 10 zusammen mit einem pharmazeutisch geeigneten und physiologisch verträglichen Trägerstoff, Zusatzstoff und/oder anderen Wirk- und Hilfsstoffen,

14. Salz gemäß einem oder mehreren der Ansprüche 1 bis 10 zur Anwendung in der Behandlung von Myokardinfarkt, Angina pectoris und andere Formen des akuten Koronarsyndroms, den Schlaganfall, die peripher vaskulären Erkrankungen, die tiefe Venenthrombose, die Lungenembolie, embolische oder thrombotische Ereignisse bedingt durch kardiale Arrhythmien, kardiovaskuläre Ereignisse wie Restenose nach Revaskularisierung und Angioplastie und ähnlichen Eingriffen wie Stentimplantationen und Bypass-Operationen handelt, oder die Reduktion der Thrombosegefahr nach chirurgischen Eingriffen wie bei Knie- und Hüftgelenksoperationen, oder um die disseminierte intravaskuläre Koagulation, Sepsis und anderen intravaskulären Ereignissen, die mit einer Entzündung einhergehen, Atherosklerose, Diabetes und dem metabolischen Syndrom und dessen Folgen, Tumorwachstum und Tumormetastasierung, Störungen des hämostatischen Systems wie Fibrinablagerungen, fibrotische Veränderungen der Lunge wie die chronische obstruktive Lungenerkrankung, das adult respiratory distress syndrome oder Fibrinablagerungen des Auges nach Augenoperationen oder Verhinderung und/oder Behandlung von Narbenbildung,

## Claims

1. A naphthalene-1,5-disulfonic acid salt of (R)-3-(6-aminopyridin-3-yl)-2-(1-cyclohexyl-1 H-imidazol-4-yl)propionic acid ethyl ester.

2. The naphthalene-1,5-disulfonic acid salt as claimed in claim 1, **characterized in that** the salt is present in crystalline form or in partially crystalline form.

3. The salt of the compound of the formula (I) as claimed in claim 1 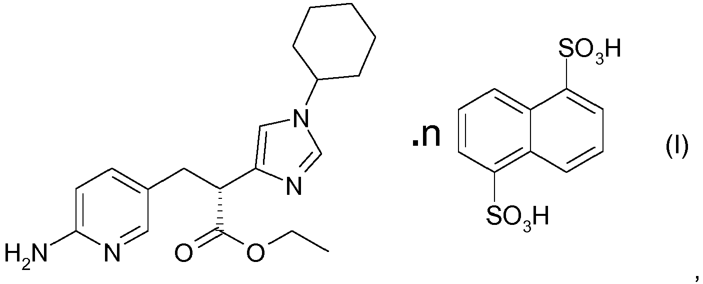 in which n has a value from 0.5 to 1.8 and n indicates the molar ratio of (R)-3-(6-aminopyridin-3-yl)-2-(1-cyclohexyl-1H-imidazol-4-yl)propionic acid ethyl ester to naphthalene-1,5-disulfonic acid.

4. The salt of the compound of the formula I as claimed in claim 3, in which n has a value from 0.8 to 1.3.

5. The salt of the compound of the formula I as claimed in claim 4, in which n has a value from 0.95 to 1.05.

6. The salt of the compound of the formula I as claimed in claim 3, **characterized in that** the XRPD measured with CuKα radiation at an angular position of 22.48 degrees 2 theta ± 0.2 degrees 2 theta has a reflection with high intensity.

7. The salt of the compound of the formula I as claimed in claim 3, **characterized in that** the XRPD measured with CuKα radiation at the angular positions has reflections at the following 2 theta values: 7.35 and 22.48 in each case ± 0.2 degrees 2 theta.

8. The salt of the compound of the formula I as claimed in claim 3, **characterized in that** the XRPD measured with CuKα radiation at the angular positions has reflections at the following 2 theta values:
7.35; 11.4; 13.69; 14.96; 17.49; 19.3 and 22.48 in each case ± 0.2 degrees 2 theta.

9. The salt of the compound of the formula I as claimed in claim 3, **characterized in that** the XRPD measured with CuKα radiation has reflections which are essentially identical to those shown in figure 1.

10. The salt of the compound of the formula I as claimed in claim 3, **characterized in that** the Raman spectrum has characteristic bands at the following wavelengths [cm⁻¹]:
2936; 1570; 1401; 1352; 998 and 856 in each case ± 2 cm⁻¹

11. A method for the production of the salt as claimed in claim 1, **characterized in that** naphthalene-1,5-disulfonic acid is dissolved in a solvent A, and a solution of (R)-3-(6-aminopyridin-3-yl)-2-(1-cyclohexyl-1 H-imidazol-4-yl)propionic acid ethyl ester in a solvent B is added, then the salt of (R)-3-(6-aminopyridin-3-yl)-2-(1-cyclohexyl-1 H-imidazol-4-yl)propionic acid ethyl ester with naphthalene-1,5-disulfonic acid of the formula (I) is obtained, optionally the solvents are partially or completely removed.

12. The production method as claimed in claim 11, **characterized in that** the solvents A and B are the same or different and are selected from the group ethanol, acetone, methyl ethyl ketone, acetonitrile, tetrahydrofuran, dimethyl sulfoxide, dimethylformamide, N-methylpyrrolidinone, methanol, propanol, butanol and water.

13. A drug **characterized by** a salt as claimed in one or more of claims 1 to 10 together with a pharmaceutically suitable and physiologically compatible carrier substance, additive and/or other active ingredients and auxiliaries.

14. The salt as claimed in one or more of claims 1 to 10 for use in the treatment of myocardial infarction, angina pectoris and other forms of acute coronary syndrome, stroke, peripheral vascular diseases, deep vein thrombosis, pulmonary embolism, embolic or thrombotic events caused by cardiac arrhythmias, cardiovascular events such as restenosis after revascularization and angioplasty and similar interventions such as stent implants and bypass operations, or the reduction in thrombosis risk following surgical interventions such as during knee and hip joint operations, or desseminated intravascular coagulation, sepsis and other intravascular events associated with inflammation, atherosclerosis, diabetes and metabolic syndrome and its consequences, tumor growth and tumor metastasization, disturbances of the hemostatic system such as fibrin deposits, fibrotic changes in the lungs, such as chronic obstructive lung disease, adult respiratory distress syndrome or fibrin deposits of the eye following eye operations or prevention and/or treatment of scarring.

## Revendications

1. Sel de l'acide naphtalène-1,5-disulfonique de l'ester éthylique de l'acide (R)-3-(6-amino-pyridin-3-yl)-2-(1-cyclohexyl-1H-imidazol-4-yl)-propionique.

2. Sel de l'acide naphtalène-1,5-disulfonique selon la revendication 1, **caractérisé en ce que** le sel se présente sous forme cristalline ou sous forme partiellement cristalline.

3. Sel du composé de formule (I) selon la revendication 1 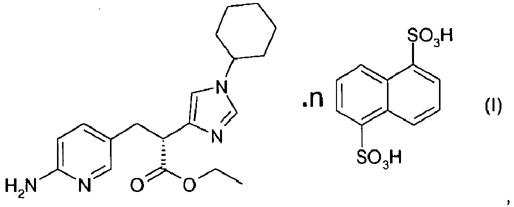 dans laquelle n a une valeur de 0,5 à 1,8, et n indique le rapport en moles de l'ester éthylique de l'acide (R)-3-(6-amino-pyridin-3-yl)-2-(1-cyclohexyl-1H-imidazol-4-yl)-propionique à l'acide naphtalène-1,5-disulfonique.

4. Sel du composé de formule I selon la revendication 3, dans lequel n a une valeur de 0,8 à 1,3.

5. Sel du composé de formule I selon la revendication 4, dans lequel n a une valeur de 0,95 à 1,05.

6. Sel du composé de formule I selon la revendication 3, **caractérisé en ce que** la diffraction aux rayons X par la méthode des poudres (XRPD), mesurée pour la radiation CuK□, présente pour une position angulaire de 22,48 degrés 2 thêta ± 0,2 degré 2 thêta une réflexion à haute intensité.

7. Sel du composé de formule I selon la revendication 3, **caractérisé en ce que** la XRPD mesurée pour la radiation CuK□ présente, pour les positions angulaires, des réflexions pour les valeurs de 2 thêta suivantes : 7,35 et 22,48, chacune ± 0,2 degré 2 thêta.

8. Sel du composé de formule I selon la revendication 3, **caractérisé en ce que** la XRPD mesurée pour la radiation CuK□ présente, pour les positions angulaires, des réflexions pour les valeurs de 2 thêta suivantes :
7,35 ; 11,4 ; 13,69 ; 14,96 ; 17,49 ; 19,3 et 22,48, chacune ± 0,2 degré 2 thêta.

9. Sel du composé de formule I selon la revendication 3, **caractérisé en ce que** la XRPD mesurée pour la radiation CuK□ présente des réflexions qui pour l'essentiel sont identiques à celles qui sont représentées sur la Figure 1.

10. Sel du composé de formule I selon la revendication 3, **caractérisé en ce que** le spectre Raman présente des bandes caractéristiques pour les longueurs d'onde suivantes [cm⁻¹] : 2936 ; 1570 ; 1401 ; 1352 ; 998 et 856, chacune ± 2 cm⁻¹.

11. Procédé de préparation du sel selon la revendication 1, **caractérisé en ce qu'**on dissout de l'acide naphtalène-1,5-disulfonique dans un solvant A, et on ajoute une solution de l'ester éthylique de l'acide (R)-3-(6-amino-pyridin-3-yl)-2-(1-cyclohexyl-1H-imidazol-4-yl)-propionique dans un solvant B, puis on obtient le sel de l'ester éthylique de l'acide (R)-3-(6-amino-pyridin-3-yl)-2-(1-cyclohexyl-1H-imidazol-4-yl)-propionique avec l'acide naphtalène-1,5-disulfonique de formule (I), éventuellement on élimine les solvants en totalité ou en partie.

12. Procédé de préparation selon la revendication 11, **caractérisé en ce que** les solvants A et B sont identiques ou différents et sont choisis dans le groupe consistant en l'éthanol, l'acétone, la méthyléthylcétone, l'acétonitrile, le tétrahydrofuranne, le diméthylsulfoxyde, le diméthylformamide, la N-méthylpyrrolidinone, le méthanol, le propanol, le butanol et l'eau.

13. Médicament, **caractérisé par** un sel selon l'une ou plusieurs des revendications 1 à 10, en même temps qu'un support, un additif et/ou d'autres principes actifs et adjuvants pharmaceutiquement appropriés et physiologiquement compatibles.

14. Sel selon l'une ou plusieurs des revendications 1 à 10, pour une utilisation lors du traitement de l'infarctus du myocarde, de l'angine de poitrine et d'autres formes du syndrome coronarien aigu, de l'accident vasculaire cérébral, des maladies vasculaires périphériques, de la thrombose veineuse profonde, de l'embolie pulmonaire, des événements emboliques ou thrombotiques consécutifs à des arythmies cardiaques, des événements cardiovasculaires tels qu'une resténose après une revascularisation et une angioplastie, et après des interventions analogues telles que l'implantation de stent et des opérations de pontage, ou pour la réduction du risque de thrombose après des interventions chirurgicales, notamment lors d'opérations de l'articulation du genou et de la hanche, ou de la coagulation intravasculaire disséminée, de la sepsie, et d'autres événements intravasculaires associés à une inflammation, de l'athérosclérose, du diabète et du syndrome métabolique et de ses séquelles, de la croissance tumorale et de la métastatisation des tumeurs, des troubles du système hémostatique tels que les dépôts de fibrine, des altérations fibrotiques des poumons, telles que la bronchopneumopathie chronique obstructive, le syndrome de détresse respiratoire de l'adulte ou les dépôts de fibrine de l'oeil après des opérations sur l'oeil, ou pour empêcher et/ou traiter la formation de cicatrices.
